# EUROPEAN PATENT APPLICATION

(11) **EP 1 862 186 A1**
(43) Date of publication of application: **05.12.2007**
(21) Application number: 06728659.1
(22) Date of filing: 06.03.2006
(51) Int. Cl.: A61L 15/64, A61L 33/00, A61L 17/00, A61B 19/00

(54) **ADHESION-PREVENTIVE FILM**

(30) Priority: 23.03.2005 JP 2005084718
(71) Applicant: JMS Co., Ltd., Hiroshima-shi, Hiroshima 730-8652 (JP)
(72) Inventor: FUJIMURA, Kenji, Hiroshima 731-0102 (JP); IDE, Junichi, Hiroshima 730-0843 (JP); MATSUURA, Yoji, Hiroshima 733-0034 (JP)
(74) Representative: Behnisch, Werner
(86) International application number: PCT/JP2006/304240
(87) International publication number: WO 2006/100895

(57) **Abstract**

An adhesion-preventive film is provided that is excellent in flexibility and can prevent cracks from occurring. The adhesion-preventive film contains a copolymer of lactide and caprolactone. The lactide and the caprolactone of the copolymer has a mole ratio in the range of 65 : 35 to 80: 20. Even when this adhesion-preventive film is used in a curved state in vivo or is wound around an affected part such as a tendon, for example, it can provide an adhesion-preventive function for a sufficiently long period without cracking.

## Description

### Technical Field

The present invention relates to an adhesion-preventive film for preventing adhesion between biological tissues.

### Background Art

In the clinical field, in order to prevent biological tissues from adhering to each other, an adhesion-preventive film is used that physically isolates an affected part from a tissue surrounding it. The adhesion-preventive film is preferably one that has suitable flexibility while exhibiting its effect during the period of time for which the adhesion-preventive effect is required and then is degraded and absorbed in vivo.

Currently, adhesion-preventive films that have been put into practical use include films made of oxidized regenerated cellulose and films formed of a mixture of hyaluronic acid and carboxymethylcellulose, for example. However, since such materials are naturally-derived materials, there are possibilities that the quality is not stable or the risk of viral infection cannot be eliminated completely. Furthermore, such materials tend to be degraded and absorbed quickly in vivo and further to be changed into a gel state and to flow when coming into contact with body fluids. Thus, adhesion-preventive films formed using such materials are used in the fields of, for example, abdominal surgery as well as obstetrics and gynecology where they are useful even if the adhesion-preventive effect lasts only for a short period of time (for example, about one week). On the other hand, it is considered that they are not suitable for the cases where isolation from the surrounding tissues is necessary for a longer period of time (for instance, at least about two weeks).

In order to solve such problems, recently, films formed of polymers or copolymers of lactic acid, lactide, caprolactone, etc. that are excellent in biocompatibility and bioabsorbability have been proposed as adhesion-preventive films (see, for example, Patent Document 1 and Patent Document 2). In addition, a process for producing a medical product from a lactide-caprolactone copolymer also has been proposed (see Patent Document 3).
[Patent Document 1: JP64(1989)-2383B]
[Patent Document 2: JP2000-189509A]
[Patent Document 3: JP3161729]

### Disclosure of Invention

### Problem to be Solved by the Invention

However, the present inventors further noticed that a lactide-caprolactone copolymer film has a suitable degradation and absorption rate but has the following problem. A lactide-caprolactone copolymer film in which the mole ratio between lactide and caprolactone is 50 : 50 has been known as a film having the best flexibility. However, although such a film is excellent in flexibility and is easy to wind around an affected part, the following has been discovered. That is, it cracks within about three weeks when it is implanted into a biological body in the state where it is wound around a tendon, for example, and therefore the tissues surrounding the tendon invade through the cracks and adhere to the tendon, and thus a satisfactory adhesion-preventive effect cannot be obtained. Particularly, since it takes time for an affected part such as a tendon to cure, it is necessary to prevent the affected part from adhering to the tissues surrounding it by isolating them from each other for about three weeks, for example. However, when cracks occur within the period described above, a satisfactory medical treatment cannot be performed.

Hence, the present invention is intended to provide an adhesion-preventive film that has suitable flexibility and can prevent cracks from occurring in use.

### Means for Solving the Problem

An adhesion-preventive film of the present invention contains a copolymer of lactide and caprolactone. The lactide and the caprolactone of the copolymer has a mole ratio in the range of 65 : 35 to 80 : 20.

### Effects of the Invention

According to the adhesion-preventive film of the present invention, the mole ratio between the lactide and the caprolactone set in the above-mentioned range allows the following effects to be provided even when the film is subjected to physical force in use as in the case where it is used in a curved state in vivo or it is wound around an affected part, for example. That is, it provides an adhesion-preventive function without cracking for a sufficiently long period and can be degraded and absorbed in vivo after the aforementioned period. Hence, it is very useful particularly as an adhesion-preventive film that is used by being wound around an affected part such as a tendon. The present inventors found out and focused on the problem for the first time that even conventional adhesion-preventive films that have excellent flexibility crack and cannot provide a satisfactory adhesion-preventive effect when they are used, for example, in a curved or wound state as described above.

### Brief Description of Drawings

FIG. 1 is a photograph showing an adhesion-preventive film according to an example of the present invention.
FIG. 2 is a photograph showing an adhesion-preventive film according to a comparative example of the present invention.

### Best Mode for Carrying Out the Invention

As described above, the present invention provides an adhesion-preventive film containing a copolymer of lactide and caprolactone. The adhesion-preventive film is characterized in that the mole ratio (A : B) between the lactide (A) and the caprolactone (B) of the copolymer is in the range of 65 : 35 to 80 : 20. With respect to the mole ratio (A : B), in the case where the proportion of caprolactone (B) becomes larger (i.e. the proportion of lactide (A) becomes smaller) than 65: 35, when it is implanted into a biological body in a curved state, it might crack before the adhesion-preventive effect is provided for a sufficiently long period, for example. On the other hand, when the proportion of caprolactone (B) becomes smaller (i.e. the proportion of lactide (A) becomes larger) than 80: 20, winding around, for instance, a tendon might be impossible per se due to the lack of flexibility, for example. Such problems might occur. The above-mentioned mole ratio (A : B) is preferably 70 : 30 to 80 : 20, preferably 75 : 25.

The weight-average molecular weight of the copolymer is not particularly limited. For example, it is 100,000 to 1,500,000, preferably 200,000 to 1,000,000, and more preferably 400,000 to 800,000. When the weight-average molecular weight is at least 100,000, the film has further improved strength, while when it is 1,500,000 or less, the copolymer is further easier to synthesize.

The copolymer of the present invention can be either a random polymer or a block polymer, for example. Furthermore, such copolymers can be used in the form of a mixture of at least two copolymers that are different from each other in the mole ratio, as long as the above-mentioned mole ratio is satisfied. The adhesion-preventive film of the present invention may contain only a copolymer having the above-mentioned mole ratio or may contain another additional polymer or copolymer as long as it does not affect the present invention.

The process for preparing a copolymer in the present invention is not particularly limited. Conventionally well-known methods can be used. Generally, lactide and caprolactone that are used as starting materials may be copolymerized through ring-opening polymerization, or lactide (cyclic dimer of lactic acid) may be synthesized from lactic acid and then this may be copolymerized with caprolactone. The temperature at which lactide and caprolactone are polymerized is not particularly limited. However, it is preferably 150 to 170°C, for example, since it allows a film having excellent flexibility to be obtained. The method of synthesizing lactide using lactic acid also is not particularly limited. Conventionally well-known methods can be used.

The aforementioned lactide is not particularly limited. It can be L-lactide, D-lactide, or a mixture thereof (D, L-lactide). In addition, the lactic acid to be used herein can be L-lactic acid, D-lactic acid, or a mixture thereof (D,L-lactic acid). When lactic acid is used as a starting material, with monomer lactic acid being expressed in terms of dimer lactide, the mole ratio between the dimer lactide and caprolactone should be in the above-mentioned ranges in the copolymer of the present invention.

Examples of caprolactone include epsilon-caprolactone, gamma-caprolactone, delta-caprolactone, etc. Among them, epsilon-caprolactone is preferable.

The average chain length that is a repeating unit of the lactide in the copolymer is preferably 10 or less and more preferably 6 or less, for example. The phrase "the average chain length that is a repeating unit of the lactide in the copolymer" denotes the average number of molecules of the lactide (2 molecules of lactic acid) ring-opened during the copolymer formation that are bonded to each other continuously. When the average chain length of the lactide is 10 or less, films to be obtained have excellent flexibility and are easy to be wound, for example. Such copolymers can be obtained with the polymerization temperature being set at a high temperature, for example. Specifically, the polymerization temperature set at a relatively high temperature allows the degree of polymerization to be relatively low. Specific preferable examples of the polymerization temperature include 150 to 170°C.

The thickness of the adhesion-preventive film of the present invention is not particularly limited. However, it is preferably in the range of 50 to 300 µm, more preferably in the range of 100 to 200 µm, and particularly preferably 100 to 150 µm, for example. A thickness of 50 µm or more allows the film to have higher strength, while a thickness of 300 µm or less allows the film to have higher flexibility. In this case, it therefore is easy to wind the film around an affected part.

The process for producing an adhesion-preventive film of the present invention is not particularly limited. It can be produced by conventionally well-known film formation processes, such as an extrusion molding method, a pressing method, a casting method, etc. In a specific example, when the pressing method is employed, pellets of the copolymer are prepared and then are pressed with a hot press to be formed into a film. Furthermore, the conditions for hot pressing also are not particularly limited but generally include a temperature of 120 to 200°C and a pressure of 1 to 10 MPa. When the casting method is employed, a polymer solution is prepared by dissolving the copolymer in a solvent and is cast onto flat surface, then the solvent is volatilized, and thereby a film is formed, for example. The solvent is not particularly limited. For example, it can be 1,4-dioxane, dimethyl carbonate, chloroform, acetone, etc. The film thus produced can be used as an adhesion-preventive film without further processing.

The adhesion-preventive film of the present invention can exhibit the adhesion-preventive function without cracking for a sufficiently long period of time even when it is used in a curved state in vivo or it is wound around, for example, a tendon as described above. Hence, the adhesion-preventive film of the present invention is highly useful, especially for applications where it is wound around or it is used in a curved state.

The method of application thereof in vivo is not particularly limited. It can be placed in an abdominal area or in areas to be treated in obstetrics and gynecology but is suitable for the application to a curved affected part, for example. Specifically, for example, in addition to the application to bones of the legs, arms, etc. and joints, it is suitable particularly for the application to affected parts whose diameters are approximately 1 to 20 mm, such as a tendon, bone, joint, blood vessel, lymphatic vessel, oviduct, etc. When a conventional adhesion-preventive film is wound around such an affected part, the film is subjected to physical force as compared to the case where the film is placed simply in an abdominal area, etc. as described above. This becomes one of the causes of cracking the film within a treatment period. However, according to the adhesion-preventive film of the present invention, even when it is applied to such an affected part in a curved state or it is applied by being wound around such an affected part, it can isolate physically the affected part satisfactorily from a tissue surrounding it without cracking during the treatment period. Specifically, when it is wound around an affected part such as a tendon, for example, it is free from cracking for around 3 to 6 weeks and then is degraded and absorbed in vivo. Generally, the period of time required for preventing adhesion of a tendon, for example, is around 3 to 4 weeks. Accordingly, it can be said that the adhesion-preventive film of the present invention provides a satisfactory adhesion-preventive effect.

Hereinafter, the present invention is described further in detail using examples and comparative examples but is not limited thereto.

### Example 1

In the presence of tin octoate (50 ppm), 100 parts by mass of L-lactide and 52.8 parts by mass of epsilon-caprolactone were allowed to react with each other at a reaction temperature of 170°C under reduced pressure for 16 hours. Thus a lactide-caprolactone copolymer was synthesized. The copolymer thus synthesized had a weight-average molecular weight (Mw) of 800,000. The mole ratio (A : B) between lactide (A) and caprolactone (B) was 75 : 25. Then pellets of the lactide-caprolactone copolymer obtained were pressed with a hot press at 140°C and 10 MPa. Thus a copolymer film with a thickness of 150 µm was obtained.

### <Measurement of Weight-Average Molecular Weight>

The lactide-caprolactone copolymer was dissolved in chloroform. Using gel permeation chromatography (GPC; the developing solvent: chloroform), the weight-average molecular weight was measured in terms of standard polystyrene.

### <Measurement of Mole Ratio of Copolymer>

Using a dry lactide-caprolactone copolymer, ¹H NMR spectra were measured. The peaks of lactide and caprolactone were considered to be around 5.2 ppm and 4.1 ppm, respectively, and the mole ratio between lactide and caprolactone was determined from the ratio between the integral values of those peaks.

The skin of a forelimb finger of a dog (beagle; with a weight of 8 kg) was incised and further a tendon sheath was incised. Thus a flexor tendon in the tendon sheath was exposed. A copolymer film (with a length of 10 mm and a width of 20 mm) was sterilized with ethylene oxide gas (EOG). Thereafter, the copolymer film was wound around the flexor tendon and then was sutured together to be fixed to the tendon. Then the incised part was sutured and was immobilized with a cast. The dog was sacrificed three weeks after the copolymer film was implanted. Then the operative site was incised along the tendon. Then the copolymer film implanted therein was removed and the state thereof was observed. The same experiment was carried out with respect to copolymer films of a total of 19 samples.

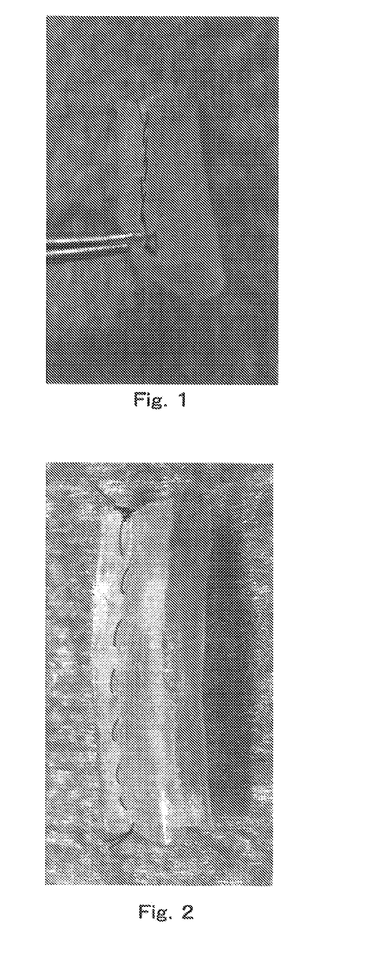

The above-mentioned copolymer films were observed visually. As a result, no cracks were observed in all the films. FIG. 1 shows the photograph of a copolymer film removed from the dog. Furthermore, the surfaces of the copolymer films were observed with a scanning electron microscope (SEM). As a result, no erosions were observed at the film surfaces even upon the SEM observation.

### Example 2

The same operation as in Example 1 was carried out (with respect to one sample) except that the copolymer used herein was one obtained by allowing 100 parts by mass of L-lactide and 79 parts by mass of epsilon-caprolactone to react with each other. The lactide-caprolactone copolymer obtained herein had a weight-average molecular weight of 500,000, while the mole ratio (A : B) between lactide (A) and caprolactone (B) was 65 : 35. As a result, no cracks were found in the film by visual observation as in the case of Example 1.

### Example 3

The same operation as in Example 1 was carried out (with respect to one sample) except that the copolymer used herein was one obtained by allowing 100 parts by mass of L-lactide and 42.6 parts by mass of epsilon-caprolactone to react with each other. The lactide-caprolactone copolymer obtained herein had a weight-average molecular weight of 450,000, while the mole ratio (A: B) between lactide (A) and caprolactone (B) was 80 : 20. As a result, no cracks were found in the film by visual observation as in the case of Example 1.

### Comparative Example 1

The same operation as in Example 1 was carried out (with respect to six samples) except that the copolymer used herein was one obtained by allowing 100 parts by mass of L-lactide and 119 parts by mass of epsilon-caprolactone to react with each other. The lactide-caprolactone copolymer obtained herein had a weight-average molecular weight of 600,000, while the mole ratio (A : B) between lactide (A) and caprolactone (B) was 50 : 50.

Each sample was observed visually. As a result, a number of cracks of approximately 1 to 20 mm were observed in all the films. Mainly, cracks had occurred along the longitudinal direction of the tendon. FIG. 2 shows the photograph of a copolymer film removed from the dog. Similar cracks to those shown in FIG. 2 were found in all the copolymer films. Furthermore, the surfaces of the films were observed with the SEM. As a result, erosions (surface roughness) were observed across the film surfaces even in the regions where no cracks had occurred. Since the copolymer films of Comparative Example 1 each had a higher ratio of caprolactone, they had higher flexibility than those of the copolymer films of the examples. However, the copolymer films of the comparative example were cracked by being wound. This result shows that a simple improvement in flexibility cannot prevent the film from cracking when it is wound.

### Comparative Example 2

The same operation as in Example 1 was carried out (with respect to one sample) except that the copolymer used herein was one obtained by allowing 100 parts by mass of L-lactide and 85.3 parts by mass of epsilan-caprolactone to react with each other. The lactide-caprolactone copolymer obtained herein had a weight-average molecular weight of 400,000, while the mole ratio (A : B) between lactide (A) and caprolactone (B) was 60 : 40. Each sample was observed visually. As a result, a number of cracks of approximately 1 to 20 mm were observed in the film of one sample as in Comparative Example 1. Mainly, cracks had occurred along the longitudinal direction of the tendon.

### Comparative Example 3

The same operation as in Example 1 was carried out except that the copolymer used herein was one obtained by allowing 100 parts by mass of L-lactide and 19.8 parts by mass of epsilon-caprolactone to react with each other. The lactide-caprolactone copolymer obtained herein had a weight-average molecular weight of 350,000, while the mole ratio (A : B) between lactide (A) and caprolactone (B) was 90 : 10. However, the film produced herein was very stiff and therefore could not be wound around a tendon.

### Example 4

A copolymer film (with a length of 10 mm and a width of 20 mm) that had been subjected to the same EOG sterilization as in Example 1 was wound around a sterilized Teflon (Registered Trademark) rod (with a diameter of 1.5 mm and a length of 20 mm) and then was sutured together with a surgical suture to be fixed closely to the above-mentioned Teflon (Registered Trademark) rod. Thus a sample was obtained. The dorsal skin of a dog (beagle with a weight of 8 kg) was incised and then the sample was implanted subcutaneously. Thereafter, the incised part was sutured. When the sample was implanted, the sample was inserted simply into a subcutaneous pocket that was created by making a slit in the dorsum of the dog and was not fixed to subcutaneous tissues or the like. The dog was sacrificed three weeks after the sample was implanted. Then the operative site was incised and the sample was removed. Thereafter the state thereof was observed (three samples).

The respective samples thus removed were observed visually. As a result, no cracks were observed in any of the films. Furthermore, the surfaces of the copolymer films were observed using the scanning electron microscope (SEM). However, no erosions of the film surfaces were found even upon the SEM observation.

### Comparative Example 4

The same operation as in Example 4 was carried out (with respect to six samples) except that the copolymer film produced in Comparative Example 1 was used. Each sample that had been removed was observed visually. As a result, a number of cracks of approximately 1 to 20 mm were observed in all the films. The cracks had occurred mainly along the longitudinal direction of the Teflon (Registered Trademark) rod around which each film was wound.

The results of visual observation for the presence of cracks in the respective examples and comparative examples are shown in Table 1 below.

**Table 1**

| | Total Number of Samples | Number of Samples with No Cracks Observed | Number of Samples with Cracks Observed |
|---|---|---|---|
| Example 1 | 19 | 19 | - |
| Example 2 | 1 | 1 | - |
| Example 3 | 1 | 1 | - |
| Comparative Example 1 | 6 | - | 6 |
| Comparative Example 2 | 1 | - | 1 |
| Comparative Example 3 | Not Examinable | - | - |
| Example 4 | 3 | 3 | - |
| Comparative Example 4 | 6 | - | 6 |

As shown in Table 1 above, when the adhesion-preventive films of the examples were used, no cracks were observed. On the other hand, in the case of the adhesion-preventive films of the comparative examples, cracks occurred with high probability. In the case of the adhesion-preventive film of Comparative Example 3, winding itself was not possible. Furthermore, from the result of Example 4, it was proved that the crack preventive effect also was effective in sites other than tendons.

Thus, the following was found. That is, when the mole ratio between lactide and caprolactone is set in a predetermined range, a copolymer film can be obtained that has suitable flexibility and can be prevented from cracking in use.

### Industrial Applicability

As described above, the adhesion-preventive film of the present invention has suitable flexibility and can be prevented from cracking in use. Accordingly, even when the film is used, for example, in a curved or wound state and thereby the film is subjected to physical force, it can exhibit the adhesion-preventive function for a sufficiently long period of time. It can be said that the adhesion-preventive film is particularly useful when it is used for a tendon which requires time for medical treatment and around which the adhesion-preventive film has to be wound.

## Claims

1. An adhesion-preventive film comprising a copolymer of lactide and caprolactone,
wherein the lactide and the caprolactone of the copolymer have a mole ratio in a range of 65 : 35 to 80 : 20.

2. The adhesion-preventive film according to claim 1, wherein the lactide and the caprolactone of the copolymer have a mole ratio in a range of 70 : 30 to 80 : 20.

3. The adhesion-preventive film according to claim 1, wherein the lactide and the caprolactone of the copolymer have a mole ratio of 75 : 25.

4. The adhesion-preventive film according to claim 1, wherein the adhesion-preventive film is a film that is used in a curved state in vivo.

5. The adhesion-preventive film according to claim 1, wherein the adhesion-preventive film is a film that is used in a wound state.

6. The adhesion-preventive film according to claim 1, wherein the adhesion-preventive film is a film to be wound around at least one region selected from the group consisting of a nerve, tendon, bone, joint, blood vessel, lymphatic vessel, and oviduct.

7. The adhesion-preventive film according to claim 1, wherein the adhesion-preventive film has a thickness in a range of 50 to 300 µm.

8. The adhesion-preventive film according to claim 1, wherein the copolymer has a weight-average molecular weight in a range of 100,000 to 1,500,000.

9. The adhesion-preventive film according to claim 1, wherein the copolymer has a weight-average molecular weight in a range of 200,000 to 1,000,000.

10. The adhesion-preventive film according to claim 1, wherein the copolymer has a weight-average molecular weight in a range of 400,000 to 800,000.

11. The adhesion-preventive film according to claim 1, wherein the lactide of the copolymer has an average chain length of 10 or less.

12. The adhesion-preventive film according to claim 1, wherein the lactide of the copolymer has an average chain length of 6 or less.
